# EUROPEAN PATENT SPECIFICATION

(11) **EP 0 719 161 B1**
(45) Date of publication and mention of the grant of the patent: **17.04.2002**
(21) Application number: 94927997.0
(22) Date of filing: 01.09.1994
(51) Int. Cl.: A61M 37/00

(54) **ENDOVASCULAR SYSTEM FOR ARRESTING THE HEART**
ENDOVASKULARES SYSTEM ZUM ERZEUGEN EINES KÜNSTLICHEN HERZSTILLSTANDS
DISPOSITIF ENDOVASCULAIRE POUR ARRETER LE COEUR

(30) Priority: 17.09.1993 US 123411
(43) Date of publication of application: 03.07.1996
(60) Divisional application: 02001320.7
(73) Proprietor: HEARTPORT, INC., Redwood City, California 94063 (US)
(72) Inventor: EVARD, Philip, C, Palo Alto, CA 94306 (US); MACHOLD, Timothy, R., Moss Beach, CA 94038 (US); STEVENS, John, H., Palo Alto, CA 94303 (US)
(74) Representative: Harrison, David Christopher
(86) International application number: US9409938
(87) International publication number: WO9508364

(56) References cited:
- US-A- 4 582 181
- US-A- 4 784 639
- US-A- 5 195 942
- US-A- 5 226 427

## Description

This invention relates generally to devices for performing cardiovascular, pulmonary and neurosurgical procedures wherein cardiac function is arrested and the patient is placed on cardiopulmonary bypass. More specifically, the invention relates to devices for isolating the heart and coronary blood vessels from the remainder of the arterial system, to facilitate arresting the heart and establishing cardiopulmonary bypass.

Various cardiovascular, neurosurgical, pulmonary and other interventional procedures, including repair or replacement of aortic, mitral and other heart valves, repair of septal defects, pulmonary thrombectomy, coronary artery bypass grafting, angioplasty, atherectomy, treatment of aneurysms, electrophysiological mapping and ablation, and neurovascular procedures, may require general anesthesia, cardiopulmonary bypass, and arrest of cardiac function. In such procedures, the heart and coronary blood vessels must be isolated from the remainder of the circulatory system. This serves several purposes. First, such isolation facilitates infusion of cardioplegic fluid into the coronary arteries in order to perfuse the myocardium and thereby arrest cardiac function, without allowing the cardioplegic fluid to be distributed elsewhere in the patient's circulatory system. Second, such isolation facilitates the use of a cardiopulmonary bypass system to maintain circulation of oxygenated blood throughout the circulatory system while the heart is stopped, without allowing such blood to reach the coronary arteries which might resuscitate the heart. Third, in cardiac procedures, such isolation creates a working space into which the flow of blood and other fluids can be controlled or prevented so as to create an optimum surgical environment.

Using current techniques, isolation of the heart and coronary blood vessels is accomplished by placing a mechanical cross-clamp externally on the ascending aorta downstream of the ostia of the coronary arteries, but upstream of the brachiocephalic artery, so as to allow oxygenated blood from the cardiopulmonary bypass system to reach the arms, neck, head, and remainder of the body. A catheter is then inserted directly into the ascending aorta between the cross-clamp and the aortic valve, and cardioplegic fluid is infused through the catheter into the ascending aorta and coronary arteries to perfuse the myocardium. An additional catheter may be introduced into the coronary sinus for retrograde perfusion of the myocardium with cardioplegic fluid. In addition, the myocardium is usually cooled by irrigating with cold saline solution and/or application of ice or cold packs to the myocardial tissue. Cardiac contractions will then cease.

Known techniques for performing major surgeries such as coronary artery bypass grafting and heart valve repair and replacement have generally required open access to the thoracic cavity through a large open wound, known as a thoracotomy. Typically, the sternum is cut longitudinally (a median sternotomy), providing access between opposing halves of the anterior portion of the rib cage to the heart and other thoracic vessels and organs. An alternate method of entering the chest is via a lateral thoracotomy, in which an incision, typically 10 cm to 20 cm in length, is made between two ribs. A portion of one or more ribs may be permanently removed to optimize access.

In procedures requiring a thoracotomy, the ascending aorta is readily accessible for placement of an external cross-clamp through this large opening in the chest. However, such surgery often entails weeks of hospitalization and months of recuperation time, in addition to the pain and trauma suffered by the patient. Moreover, while the average mortality rate associated with this type of procedure is about two to fifteen per cent for first-time surgery, mortality and morbidity are significantly increased for reoperation. Further, significant complications may result from such procedures. For example, application of an external cross-clamp to a calcified or atheromatous aorta may cause the of release of emboli into the brachiocephalic, carotid or subclavian arteries with serious consequences such as strokes. In up to 6% of the open-chest coronary bypass surgeries performed in the United States, there is noticeable mental deterioration which is commonly attributed to cerebral arterial blockage from emboli released during the bypass procedure.

New devices are therefore desired which facilitate the performance of cardiac procedures such as heart valve repair and replacement, coronary artery bypass grafting, and the like, using minimally invasive techniques, eliminating the need for a thoracotomy, and desirably, eliminating the need for an external aortic cross-clamp. Such techniques have been described in US-A-5370685 (application Serial No. 07/730,559). This discloses methods of performing endovascular heart valve replacement in which a malfunctioning or diseased valve may be removed and replaced by means of endovascular instruments introduced transluminally through a femoral artery, iliac artery and the aorta without the need for a thoracotomy. Similarly, in commonly-assigned US-A-5452733 (application serial No. 08/023,778) methods and devices are described for performing coronary bypass grafting and other procedures through small incisions or cannulae positioned through the chest wall, obviating the need for a thoracotomy.

This new generation of minimally-invasive cardiac procedures provides significant advantages over current open surgical techniques, including reduced mortality and morbidity, decreased patient suffering, reduced hospitalization and recovery time, and lowered medical costs relative to open-chest procedures. An important factor in the ability to carry out these procedures, however, is the ability to arrest the heart and establish cardiopulmonary bypass without a thoracotomy for open access to the heart and thoracic vessels.

Devices are therefore needed for isolating the heart and coronary arteries from the remainder of the arterial system, arresting cardiac function and establishing cardiopulmonary bypass without the open-chest access provided by a thoracotomy. Further, the devices should facilitate such isolation of the heart and coronary arteries without the high risk of embolus production associated with external aortic cross-clamps.

In US-A-5195942 an endovascular resuscitation device is disclosed. A balloon on a catheter is placed in the ascending aorta and is alternately inflated to block the aorta and deflated. While it is inflated, oxygen-rich blood is injected past it through a tube which passes through it.

In contrast, the present invention provides endovascular devices as described in claim 2 for partitioning a patient's ascending aorta between the coronary ostia and the brachiocephalic artery to isolate the heart and coronary arteries from the remainder of the arterial system, arrest cardiac function, and establish cardiopulmonary bypass. The invention further provides a system as described in claim 12 for arresting the heart that facilitate isolating the heart and coronary arteries from the remainder of the arterial system, arresting cardiac function, and establishing cardiopulmonary bypass without the need for a thoracotomy or an external aortic cross-clamp.

Using the device and system of the invention, all blood flow through the ascending aorta may be blocked and cardioplegic fluid may be introduced through the coronary arteries to perfuse the myocardium. With the patient connected to cardiopulmonary bypass equipment to maintain circulation of oxygenated blood while the heart is stopped, surgical procedures may be performed on the heart, coronary blood vessels and other body structures using thoracoscopic and/or endovascular tools, without the need for a thoracotomy. Moreover, by partitioning the aorta by endovascular occlusion rather than by external cross-clamping, the device of the invention may substantially reduce the risk of embolus release associated with such cross-clamping.

For purposes of the present application, "downstream" means in the direction of normal blood flow through a blood vessel, i.e., further from the heart in the arterial system, and closer to the heart in the venous system. "Upstream" means in the direction opposite the downstream direction. With respect to devices, "proximal" means in the direction toward the end of the device that is closest to and held or manipulated by the user, while "distal" means in the direction away from the user, opposite the proximal direction.

In a particular aspect of the invention, an endovascular device for partitioning the ascending aorta between the coronary ostia and the brachiocephalic artery comprises a flexible shaft having a distal end, a proximal end, and a first inner lumen therebetween with an opening at the distal end in communication with the first inner lumen. The shaft has a distal portion which is shaped so as to be positionable within the aortic arch such that the distal end is disposed in the ascending aorta pointing toward the aortic valve. Preferably, the distal portion will be shaped so that the distal end of the shaft is spaced apart from any interior wall of the aorta, and particularly, so that the distal end is aligned with the center of the aortic valve. Expandable means are disposed near the distal end of the shaft proximal to the opening at the distal end for occluding the ascending aorta between the coronary ostia and the brachiocephalic artery, thereby blocking substantially all systolic and diastolic blood flow. The first inner lumen of the shaft may be used to withdraw blood or other fluids from the ascending aorta, to introduce cardioplegic fluid into the coronary arteries for paralyzing the myocardium, and/or to introduce surgical instruments into the ascending aorta, the coronary arteries, or the heart for performing cardiac procedures.

By "shaped," it is meant that the distal portion of the shaft is preset in a permanent, usually curved or bent shape in an unstressed condition to facilitate positioning the distal portion within at least a portion of the aortic arch, or that such a shape is imparted to the distal portion of the shaft by means of a shaping or deflecting element positioned over or within the shaft, as described in detail below.

In a preferred embodiment, the distal portion of the shaft is preshaped so as to have a generally U-shaped configuration in an unstressed condition. The U-shaped distal portion has a curvature corresponding to the curvature of the patient's aortic arch, usually having a radius of curvature in a range of 20 to 80 mm. In this way, when the preshaped distal portion is positioned in the aortic arch, the distal end will be disposed in the ascending aorta spaced apart from the interior wall thereof. Alternatively, the distal portion may have straight or curved segments with bends of relatively small radius between each segment to achieve a general "U" shape. The bends and/or segments of the preshaped distal portion may be configured to engage the interior wall of the aortic arch to deflect the distal end into a desired position in the ascending aorta. In another embodiment, the preshaped distal portion may be "S"-shaped to facilitate positioning from a location superior to the aortic arch, such as through the brachial or carotid arteries and the brachiocephalic artery.

The preshaped distal portion of the shaft may further have a distal segment which is positioned in the ascending aorta and a proximal segment which is positioned in the descending aorta, wherein the distal segment is skewed (non-coplanar) relative to the proximal segment. Such a configuration mirrors the orientation of the ascending aorta relative to the aortic arch and descending aorta, facilitating more accurate placement of the distal end in the ascending aorta, spaced apart from the interior wall thereof, and preferably, aligned with the center of the aortic valve.

The invention preferably- includes means in the shaft for straightening the preshaped distal portion. Usually, the straightening means comprises a straightening element slidably disposed in the first inner lumen having a stiffness greater than the stiffness of the preshaped distal portion. The straightening element may comprise a relatively stiff portion of a flexible guidewire extending through the first inner lumen, or a stylet having an axial passage through it for receiving a movable guidewire.

Preferably, the shaft has a bending stiffness selected to maintain the position of the occluding means against systolic blood flow from the patient's heart when the occluding means is expanded. Usually, the shaft has a bending modulus in a range of 482 to 690 MPa (70 to 100 kpsi).

In an exemplary embodiment, the occluding means has a collapsed profile for insertion into an artery such as a femoral and an expanded profile for occluding the ascending aorta, with the expanded profile diameter being about 2 to 10 times, and preferably 5 to 10 times, the collapsed profile diameter. In a preferred embodiment, the occluding means comprises an inflatable balloon, preferably of a polyurethane or polyurethane/polyvinyl blend. In one embodiment, the balloon has a blow-up ratio (defined as the ratio of the inflated outside diameter to the deflated outside diameter before collapsing) in a range of 200%-400%, and includes at least one pleat or fold when deflated which allows the balloon to collapse to an even smaller collapsed profile. Through the use of such pleats or folds, a moderately compliant material may be used to maintain balloon shape and position under the conditions present in the ascending aorta, while accommodating a range of aortic diameters. The balloon is further configured to maximize contact with the aortic wall to resist displacement and prevent leakage around the balloon, preferably having a working surface for contacting the aortic wall with a length in the range of about 3 to about 7 cm when the balloon is expanded to fully occlude the vessel.

Where a balloon is used for the occluding means, the endovascular device has an inflation lumen extending through the shaft from the proximal end to the interior of the balloon, and means connected to the proximal end of the inflation lumen for delivering an inflation fluid to the interior of the balloon. In one embodiment, the inflation fluid is a liquid such as a saline solution with a radiographic contrast agent. In a particular preferred embodiment, the inflation fluid delivery means and the inflation lumen are configured to inflate the balloon in less than about 0.5 seconds. Usually, in this embodiment, the inflation fluid is a gas such as carbon dioxide or helium. In this way, the balloon may be fully inflated between systolic contractions of the heart, reducing the likelihood of balloon displacement caused by high pressure blood flow during systole.

The shaft of the endovascular device of the invention may have a variety of configurations. The first inner lumen and inflation lumen may be coaxial, or a multi-lumen design may be employed. The shaft may further include a third lumen extending from the proximal end to the distal end of the shaft, allowing pressure distal to the occluding means to be measured through the third lumen. The shaft may also include means for maintaining the transverse dimensions of the first inner lumen, which may comprise a wire coil or braid embedded in at least the distal portion of the shaft to develop radial rigidity without loss of longitudinal flexibility. The shaft preferably has a soft tip at its distal end to prevent damage to the heart valve if the catheter comes into contact with the delicate valve leaflets.

The shaft preferably has a length of at least about 80 cm, usually about 90-100 cm, to allow transluminal positioning of the shaft from the femoral and iliac arteries to the ascending aorta. Alternatively, the shaft may have a shorter length, e.g. 20-60 cm, for introduction through the iliac artery, through the brachial artery, through the carotid artery, or through a penetration in the aorta itself.

In a particular embodiment, the first inner lumen in the shaft is configured to allow introduction of surgical or visualization instruments through the lumen for performing cardiac procedures upstream of the occluding means. In this embodiment, the first inner lumen preferably has a diameter of at least about 5 mm.

The endovascular device of the invention is particularly advantageous in that it is readily positionable in the ascending aorta, resists displacement caused by systolic blood flow, and maintains its position spaced apart from the aortic wall and axially aligned with the center of the aortic valve. The endovascular device is long enough and flexible enough to traverse the path through the femoral artery, iliac artery, descending aorta and aortic arch. At the same time, the device has sufficient pushability to be endovascularly introduced through the femoral and iliac arteries and advanced to the ascending aorta by pushing on the proximal end. Moreover, the device has sufficient axial, bending, and torsional stiffness to allow the physician to control the position of the occluding member from the proximal end, even when the device is in a tortuous vascular structure.

Because of its proximity to the left ventricle, the occluding means of the device is subject to significant forces from the outflow of blood during systole. Such forces could threaten to displace the occluding means either downstream where it might occlude the ostium of the brachiocephalic or other artery, or upstream (in a recoil effect) where the occluding means might damage the aortic valve or occlude the coronary ostia. Advantageously, the endovascular device of the invention is configured to maintain the position of the occluding means in the ascending aorta against the force of systolic outflow as the occluding means is expanded and retracted, as well as during the period in which the occluding means fully occludes the aorta but the heart remains beating.

In addition, the shaped distal portion of the device maintains the distal end in a radial position spaced apart from the interior wall of the ascending aorta such that the distal opening is unobstructed and generally aligned with the center of the aortic valve. This facilitates aspiration of blood, other fluids, or debris, infusion of fluids, or introduction of instruments through the distal opening in the endovascular device without interference with the aortic wall or aortic valve tissue.

In a further preferred embodiment, the invention provides a system for selectively arresting the heart which includes an endovascular aortic partitioning device as just described, along with means for paralyzing the patient's myocardium. Usually, the means for paralyzing the myocardium comprises means connected to the proximal end of the shaft for delivering cardioplegic fluid through the first inner lumen and out of the opening at the distal end of the device upstream of the occluding means. In this way, the occluding means may be expanded to stop blood flow through the ascending aorta, and cardioplegic fluid may be delivered through the first inner lumen to the aortic root and the coronary arteries to perfuse myocardial tissue, thereby arresting the heart. The system may further include a cardiopulmonary bypass system having means for withdrawing blood from a venous location upstream of the heart, means for oxygenating the withdrawn blood, and means for directing the oxygenated blood to an arterial location downstream of the occluding means.

In use, the distal end of the shaft of the endovascular partitioning device is introduced into a blood vessel downstream of the patient's aortic arch. The shaft is transluminally positioned so that the distal end is in the ascending aorta and the expandable occluding member attached to the shaft near the distal end is disposed between the coronary ostia and brachiocephalic artery. The occluding member is then expanded within the ascending aorta to completely block blood flow therethrough for a plurality of cardiac cycles.

The preshaped distal portion is straightened to facilitate introduction into the blood vessel, usually by positioning a stylet or guidewire in an inner lumen in the shaft. The stylet may be withdrawn from the shaft as the distal portion is advanced into the ascending aorta to allow the distal portion to resume its preshaped configuration.

Preferably, the shaft of the partitioning device is introduced through a femoral or iliac artery, brachial artery, carotid artery or other artery which is subcutaneously accessible without a thoracotomy. In this way, the device may be introduced and advanced into position with the patient's sternum and rib cage intact.

When the occluding member is an inflatable balloon, an inflation fluid is delivered to the balloon through an inner lumen in the shaft of the device. The inflation fluid may be either a liquid or a gas, and may be delivered at a rate to completely occlude the aorta between systolic contractions of the heart, usually in less than about 0.5 second.

The patient's myocardium may be paralyzed while the occluding means is expanded in the ascending aorta. Usually, this will be accomplished by infusing cardioplegic fluid through an inner lumen in the shaft of the partitioning device into the ascending aorta upstream of the occluding member. The cardioplegic fluid perfuses the myocardium through the coronary arteries to arrest heart contractions. In this case, the operation further includes the steps of withdrawing blood from a venous location upstream of the patient's heart, oxygenating the withdrawn blood, and directing the oxygenated blood to an arterial location downstream of the occluding member, thereby maintaining circulation of oxygenated blood throughout the remainder of the patient's arterial system.

With the partitioning device in position, the heart and coronary arteries isolated from the remainder of the arterial system, and the heart stopped, various diagnostic and interventional procedures may be performed. For example, instruments may be introduced for repairing or replacing the aortic or mitral valve. In this case, the operation will include the step of aligning the distal end of the shaft with the center of the aortic valve to facilitate introduction of instruments through the inner lumen of the shaft into the ascending aorta and between the valve leaflets into the left ventricle of the heart.

Thus, using the system of the invention, a patient's heart can be arrested and the patient placed on cardiopulmonary bypass without a thoracotomy, thereby reducing mortality and morbidity, decreasing patient suffering, reducing hospitalization and recovery time, and lowering medical costs relative to previous open-chest procedures. The endovascular partitioning device of the invention permits blood flow through the ascending aorta to be completely blocked between the coronary ostia and the brachiocephalic artery in order to isolate the heart and coronary arteries from the remainder of the arterial system. This has significant advantages over the aortic cross-clamps used in current cardiac procedures, not only obviating the need for a thoracotomy, but providing the ability to stop blood flow through the aorta even when calcification or other complications would make the use of an external cross-clamp undesirable.

With the endovascular partitioning device in place, the heart arrested and cardiopulmonary bypass established, the patient is prepared for a variety of surgical and diagnostic procedures, including repair or replacement of aortic, mitral and other heart valves, repair of septal defects, pulmonary thrombectomy, coronary artery bypass grafting, angioplasty, atherectomy, electrophysiological mapping and ablation, treatment of aneurysms, as well as neurovascular and neurosurgical procedures. While such procedures may be performed through a thoracotomy in the conventional manner, the invention provides the capability for performing procedures such as heart valve replacement or coronary artery bypass grafting using minimally-invasive techniques, either by means of surgical tools introduced endovascularly through the partitioning device itself, or by means of thoracoscopic tools introduced through small incisions in the chest wall.

A further understanding of the nature and advantages of the invention may be realized by reference to the remaining portions of the specification and the drawings.

Figure 1 is a side elevational view of an endovascular device for partitioning the ascending aorta between the coronary ostia and brachiocephalic artery constructed in accordance with the principles of the present invention.

Figure 1A is an end view of a distal portion of the device of Figure 1 illustrating the skew of the shaped distal portion.

Figures 1B and 1C are side elevational views showing alternative embodiments of the shaped distal portion of the device of Figure 1.

Figure 2A is a perspective view of a distal portion of the device of Figure 1 in a first embodiment thereof.

Figure 2B is a perspective view of a distal portion of the device of Figure 1 in a second embodiment thereof.

Figures 3 and 4 are transverse cross-sections taken along lines 3-3 and 4-4 in Figures 2A and 2B, respectively.

Figures 5A and 5B are transverse cross-sections taken along line 5-5 in Figure 2A, showing alternative embodiments of the shaft of the device illustrated therein.

Figure 6 is a transverse cross section taken along line 6-6 in Figure 2B.

Figure 7 is a front view of a portion of a patient's arterial system illustrating the introduction and advancement of the device of Figure 1 in the femoral artery, iliac artery and aorta.

Figure 8 schematically illustrates a system for arresting the heart constructed in accordance with the principles of the present invention, wherein the device of Figure 1 is positioned in the ascending aorta with cardioplegic fluid delivery means connected to the proximal end and a cardiopulmonary bypass system connected to the patient.

Figure 9 illustrates the distal portion of the device of Figure 1 positioned in the ascending aorta with the occluding means expanded and a tissue cutting device extended from the distal end.

The invention provides an endovascular device for partitioning the ascending aorta, as well as a system for selectively arresting the heart, which are useful in performing a variety of cardiovascular, pulmonary, neurosurgical, and other procedures. The invention is especially useful in conjunction with minimally-invasive cardiac procedures such as those described in US-A-5370685 (application Serial No. 07/730,559) and US-A-5 452 733 (U.S. patent application Serial No. 08/023,778), which are assigned to the assignee of the present invention. Procedures with which the invention may find use include repair or replacement of aortic, mitral, and other heart valves, repair of septal defects, pulmonary thrombectomy, electrophysiological mapping and ablation, coronary artery bypass grafting, angioplasty, atherectomy, treatment of aneurysms, as well as neurovascular and neurosurgical procedures. The invention is particularly advantageous in that it allows the heart to be arrested and the patient to be placed on cardiopulmonary bypass using only endovascular devices, obviating the need for a thoracotomy or other large incision. Moreover, even in conventional open-chest procedures, the endovascular aortic partitioning device of the invention will frequently find use where an external cross-clamp would raise substantial risks of embolus release due to calcification or other aortic conditions.

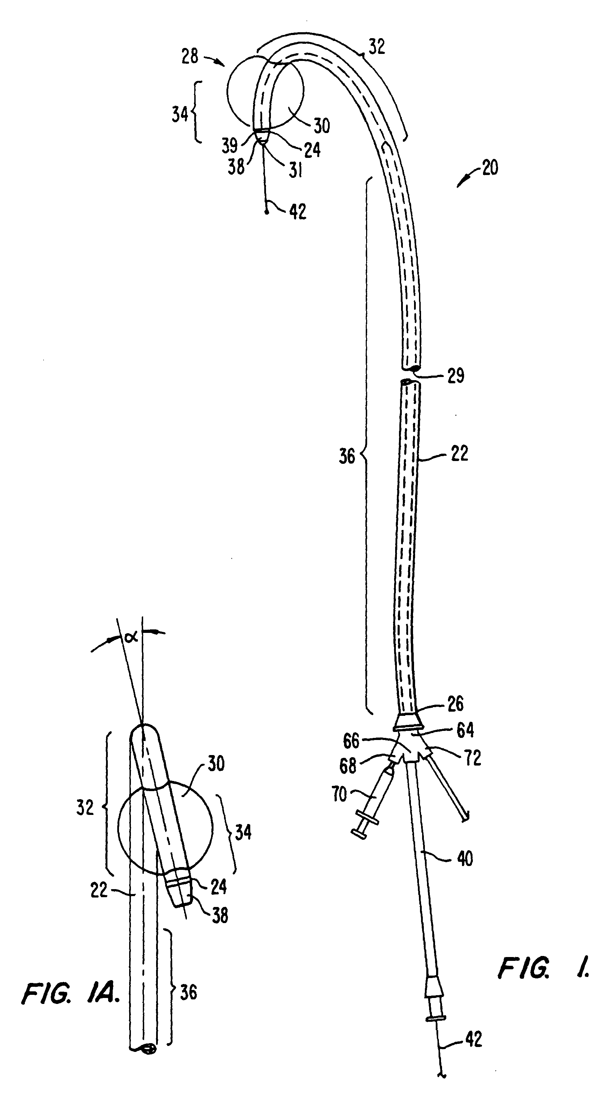

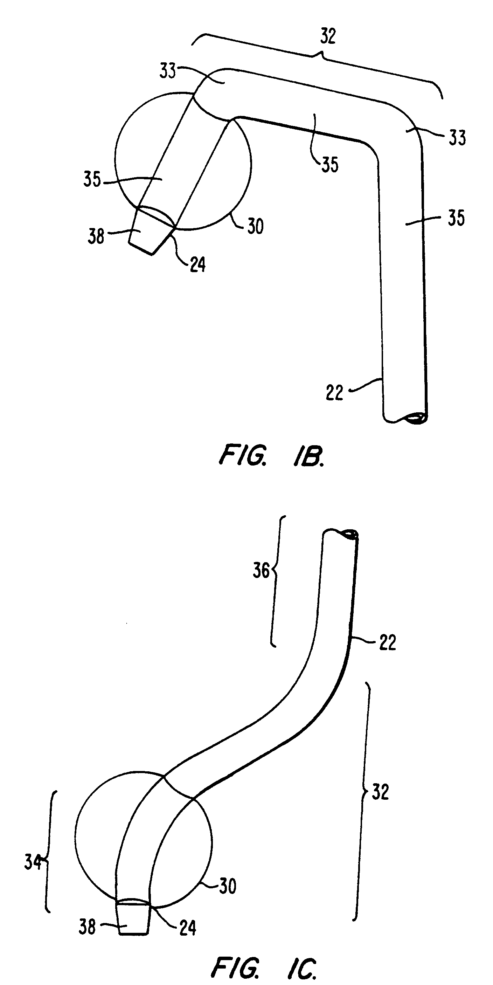

Turning now to the figures, a first preferred embodiment of an endovascular device for partitioning the ascending aorta according to the invention will be described. As illustrated in Figure 1, partitioning device 20 includes a shaft 22 having a distal end 24 and a proximal end 26. An expandable means 28 for occluding the ascending aorta is mounted to shaft 22 near distal end 24. In a preferred embodiment, occluding means 28 comprises a polymeric balloon 30 (shown inflated) of a material, geometry, and dimensions suitable for completely occluding the ascending aorta to block systolic and diastolic blood flow, as described more fully below.

Shaft 22 has a diameter suitable for introduction through a femoral or iliac artery, usually less than about 9 mm. The length of shaft 22 is preferably greater than about 80 cm, usually about 90-100 cm, so as to position balloon 30 in the ascending aorta between the coronary ostia and the brachiocephalic artery with proximal end 26 disposed outside of the body, preferably from the femoral or iliac artery in the groin area. Alternatively, the shaft may be configured for introduction through the carotid artery, through the brachial artery, or through a penetration in the aorta itself, wherein the shaft may have a length in the range of 20 to 60 cm.

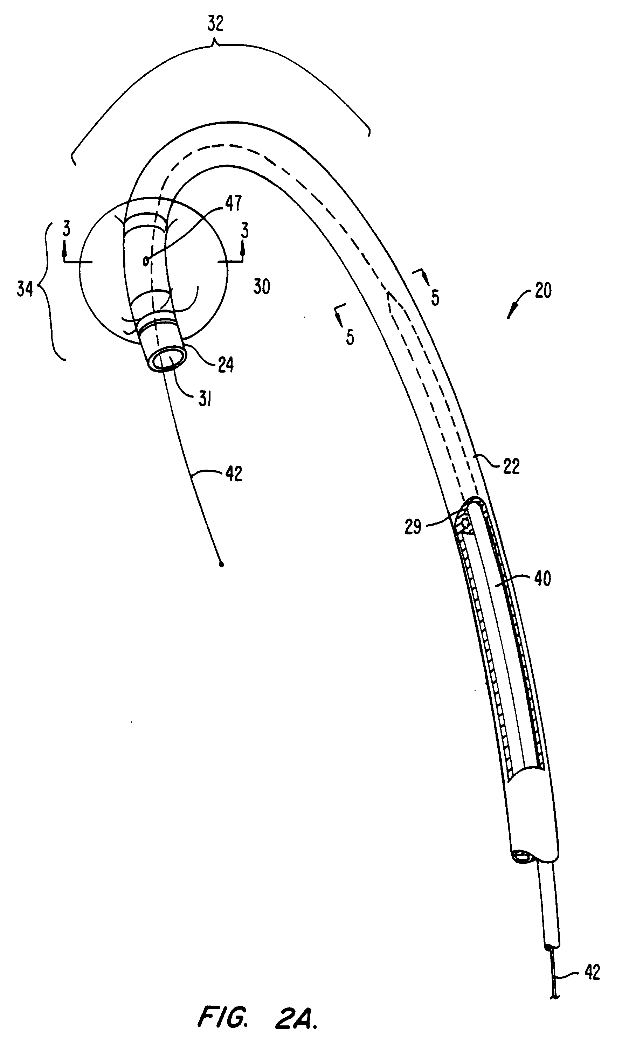

Partitioning device 20 further includes a first inner lumen 29, shown in Figures 2A-2B, extending between proximal end 26 and distal end 24 with an opening 31 at distal end 24. Additional openings in communication with inner lumen 29 may be provided on a lateral side of shaft 22 near distal end 24.

Shaft 22 has a shaped distal portion 32 configured to conform generally to the curvature of the aortic arch such that opening 31 at distal end 24 is spaced apart from the interior wall of the aorta and is axially aligned with the center of the aortic valve. Usually, shaped distal portion 32 will be generally U-shaped, such that a distal segment 34 is disposed at an angle between 135° and 225°, and preferably at approximately 180° relative to an axial direction defined by the generally straight proximal segment 36 of shaft 22. Shaped distal portion 32 will usually have a radius of curvature in the range of 20-80 mm (measured at the radial center of shaft 22), depending upon the size of the aorta in which the device is used. The configuration of shaped distal portion 32 allows distal segment 34 to be positioned centrally within the lumen of the ascending aorta and distal end 24 to be axially aligned with the center of the aortic valve, thereby facilitating infusion or aspiration of fluids as well as introduction of surgical tools through opening 31 without interference with the wall of the aorta, as described more fully below.

In an exemplary embodiment, shaped distal portion 32 is preshaped so as to maintain a permanent, generally U-shaped configuration in an unstressed condition. Such a preshaped configuration may be formed by positioning a mandrel having the desired shape in first inner lumen 29, then baking or otherwise heating shaft 22 and the mandrel for a sufficient time and sufficient temperature to create a permanent set therein, e.g., 1-3 hours at a temperature in a range of 120°C to 180°C, depending upon the material used for shaft 22.

Alternative embodiments of shaped distal portion 32 are illustrated in Figures 1B and 1C. In the embodiment of Figure 1B, U-shaped distal portion 32, rather than having a continuous, constant curvature, is preshaped in a more angular fashion, with bends 33 of relatively small curvature separating segments 35 which are either straight or of larger curvature. Bends 33 and/or segments 35 may further be configured to engage the inner wall of the aorta along the aortic arch to deflect distal end 24 into a desired position in the ascending aorta. Such engagement with the aortic wall also prevents downstream migration of balloon 30 within the aorta, which poses the risk of occluding the ostia of the brachiocephalic, left common carotid, or left subclavian arteries.

In the embodiment of Figure 1C, shaped distal portion 32 is configured in a general "S" shape for introduction into the ascending aorta from a location superior to the aortic arch. In this way, distal segment 34 may be positioned within the ascending aorta, with proximal segment 36 extending from the aortic arch through the brachiocephalic artery to the carotid or brachial artery, or through a penetration in the aorta itself, to a point outside of the thoracic cavity.

As shown in Figure 1A, distal segment 34 may be skewed (non-coplanar) relative to a central longitudinal axis of proximal segment 36, in order to further conform to the shape of the patient's aortic arch and align with the center of the aortic valve. In an exemplary embodiment, distal segment 34 is disposed at an angle α relative to a plane containing the central axis of proximal portion 36, wherein α is between 2° and 30°, usually between 10° and 20°, and preferably about 15°. The shape and dimensions of shaped distal portion 32 and angle α of distal segment 34 may vary, however, according to the configuration of the aortic arch in any individual patient.

In a preferred embodiment, the device will include a soft tip 38 attached to distal end 24 to reduce the risk of damaging cardiac tissue, particularly the leaflets of the aortic valve, in the event the device contacts such tissue. Soft tip 38 may be straight or tapered in the distal direction, with an axial passage aligned with opening 31 at the distal end of shaft 22. Preferably, soft tip 38 will be a low durometer polymer such as polyurethane or Pebax, with a durometer in the range of 65 Shore A to 35 Shore D.

At least one radiopaque stripe or marker 39 is preferably provided on shaft 22 near distal end 24 to facilitate fluoroscopic visualization for positioning balloon 30 in the ascending aorta. Radiopaque marker 39 may comprise a band of platinum or other radiopaque material. Alternatively, a filler of barium or bismuth salt may be added to the polymer used for shaft 22 or soft tip 38 to provide radiopacity.

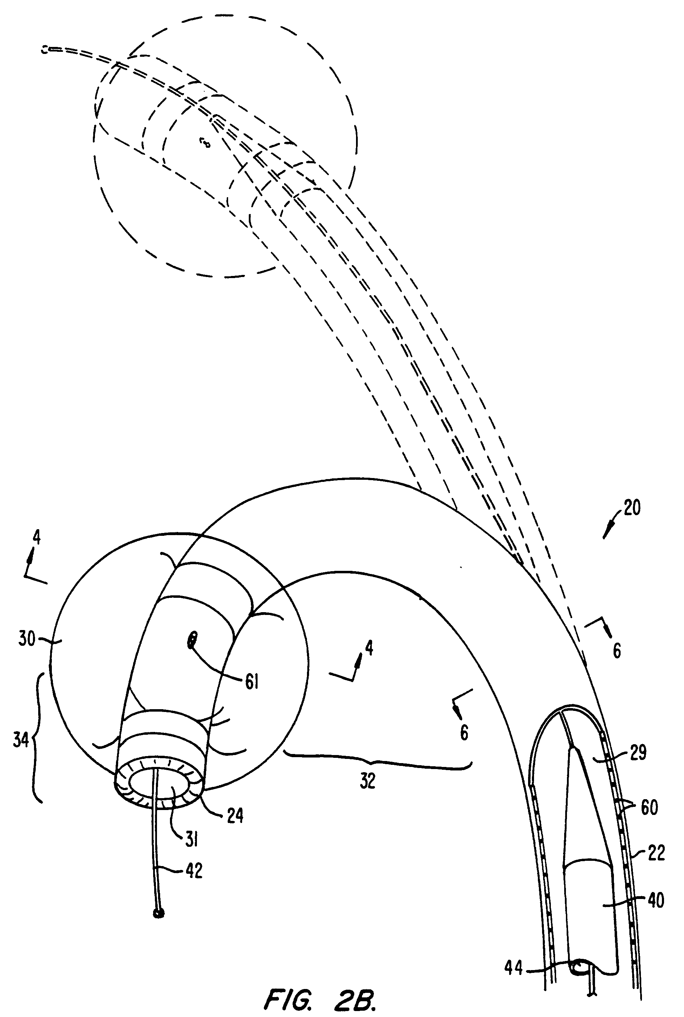

As illustrated in Figures 1, 2A and 2B, a straightening element 40 is disposed in first inner lumen 29 of shaft 22 so as to slide longitudinally relative to the shaft. Straightening element 40 may comprise a tubular stylet with a longitudinal passage 44 for receiving a guidewire 42, as described below. Alternatively, element 40 may comprise a relatively stiff portion of the guidewire itself. Straightening element 40 may be a polymeric material or a biocompatible metal such as stainless steel or nickel titanium alloy with a bending stiffness greater than that of shaft 22. In this way, straightening element 40 may be advanced distally into preshaped distal portion 32 so as to straighten shaft 22, facilitating subcutaneous introduction of partitioning device 20 into an artery and advancement to the aortic arch. Straightening element 40 may then be retracted proximally relative to the shaft so that distal end 24 can be positioned in the ascending aorta with preshaped distal portion 32 conforming to the shape of the aortic arch.

A movable guidewire 42 is slidably disposed through first inner lumen 29, either through longitudinal passage 44 in straightening element 40 (Figure 2B), external and parallel to straightening element 40, or through a separate lumen (not shown) in shaft 22. Guidewire 42 extends through opening 31 in distal end 24 of shaft 22 and may be advanced into an artery distal to shaft 22, facilitating advancement of shaft 22 through the artery to the ascending aorta by sliding the shaft over the guidewire. In an exemplary embodiment, guidewire 42 is relatively stiff so as to at least partially straighten shaft 22, so that straightening element 40 is unnecessary for introduction of shaft 22. In this embodiment, guidewire 42 may be, for example, stainless steel or a nickel titanium alloy with a diameter of about 1.0 mm to 1.6 mm.

Shaft 22 may have any of a variety of configurations depending upon the particular procedure to be performed. In one embodiment, shaft 22 has a multi-lumen configuration with three non-coaxial parallel lumens in a single extrusion, as illustrated in Figures 2A, 3 and 5A. The three lumens include first inner lumen 29, which receives straightening element 40 and guidewire 42 and includes opening 31 at its distal end, an inflation lumen 46 which opens at an inflation orifice 47 near the distal end of shaft 22 in communication with the interior of balloon 30, and a third lumen 48 which has an opening (not shown) at distal end 24 of the shaft to sense pressure in the ascending aorta. In this embodiment, the largest transverse dimension of first inner lumen 29 is preferably about 1mm-4mm. Advantageously, the distal opening in third lumen 48 is radially offset from opening 31 in first inner lumen 29, so that infusion or aspiration of fluid through first inner lumen 29 will not affect pressure measurements taken through third lumen 48.

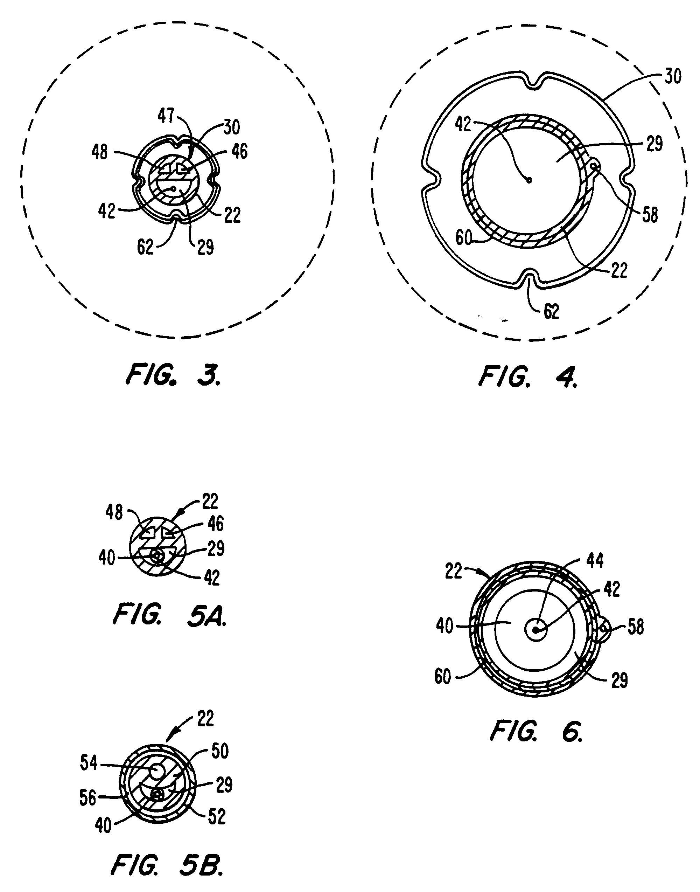

In a second embodiment, illustrated in Figure 5B, shaft 22 has a dual lumen inner member 50 and a coaxial outer member 52. Inner member 50 includes first inner lumen 29 which receives straightening element 40 and opens at distal opening 31, and a third lumen 54 which has an opening (not shown) at its distal end for measuring pressure in the ascending aorta. Outer member 52 defines a coaxial inflation lumen 56 which, at its distal end, is in communication with the interior of balloon 30. Balloon 30 and outer member 52 may comprise a single integrated extrusion, or balloon 30 may be bonded or otherwise attached to outer member 52 near the distal end of shaft 22 using well-known techniques. Outer member 52 may have an open distal end which communicates with the interior of balloon 30. Alternatively, the distal end of outer member 52 may be closed, for example, by bonding to the exterior of inner member 50, with an inflation orifice 47 provided as shown in Fig. 2A for communication between lumen 56 and the interior of the balloon.

In a third embodiment, illustrated in Figures 2B, 4 and 6, shaft 22 has a first inner lumen 29 of large diameter configured to receive various types of surgical instruments, as well as to receive straightening element 40. An inflation lumen 58 extends parallel to first inner lumen 29 and is in communication with the interior of balloon 30 through an inflation orifice 61, shown in Figure 2B. In this embodiment, shaft 22 may comprise a single extrusion containing inflation lumen 58 and inner lumen 29, or two individual tubes bonded to one another, one tube containing lumen 29 and the other containing inflation lumen 58. With this construction, shaft profile can be minimized while making lumen 29 as large as possible within the confines of the vessels in which the device is positioned. In this embodiment, first inner lumen 29 will have a diameter of at least about 5 mm and preferably about 8 mm. Partitioning device 20 thereby provides a passage of maximum diameter for endovascular introduction of surgical instruments such as visualization scopes, aspirators, irrigation tubes, cutting, stapling and suturing devices.

It should be noted that where partitioning device 20 is to be utilized for antegrade delivery of cardioplegic fluid through first inner lumen 29, it will be configured to provide a sufficient flowrate of such fluid to maintain paralysis of the heart, while avoiding undue hemolysis in the blood component (if any) of the fluid. In a presently preferred embodiment, cold blood cardioplegia is the preferred technique for arresting the heart, wherein a cooled mixture of blood and a crystalloid KCl/saline solution is introduced into the coronary arteries to perfuse and paralyze the myocardium. The cardioplegic fluid mixture is preferably run through tubing immersed in an ice bath so as to cool the fluid to a temperature of about 3°C - 10°C prior to delivery through inner lumen 29. The cardioplegic fluid is delivered through inner lumen 29 at a sufficient flowrate and pressure to maintain a pressure in the aortic root (as measured through third lumen 48) high enough to induce flow through the coronary arteries to perfuse the myocardium. Usually, a pressure of about 6,67-13,33 KPa (50-100 mmHg), preferably 8,00-9,33 KPa (60-70 mmHg) is maintained in the aortic root during infusion of cardioplegic fluid, although this may vary somewhat depending on patient anatomy, physiological changes such as coronary dilation, and other factors. At the same time, in pumping the cardioplegic fluid through inner lumen 29, it should not be subject to pump pressures greater than about 40,10 KPa (300 mmHg), so as to avoid hemolysis in the blood component of the fluid mixture. In an exemplary embodiment, first inner lumen 29 is configured to facilitate delivery of the cardioplegic fluid at a rate of about 250-350 ml/min. preferably about 300 ml/min., under a pressure of no more than about 300 ml/min, enabling the delivery of about 500-1000 ml of fluid in 1-3 minutes. To provide the desired flowrate at this pressure, inner lumen 29 usually has a cross-sectional area of at least about 4.5 mm², and preferably about 5.6-5.9 mm². In an exemplary embodiment, D-shaped lumen 29 in Fig. 5A has a straight wall about 3.3 mm in width, and a round wall with a radius of about 1.65 mm. A completely circular lumen 29 (not pictured), could have an inner diameter of about 2.7 mm. Inner lumen 29 could be significantly smaller, however, if the cardioplegic fluid did not have a blood component so that it could be delivered under higher pressures without risk of hemolysis. Because of its myocardial protective aspects, however, the forementioned blood/KCl mixture is presently preferred, requiring a somewhat larger lumen size than would be required for a crystalloid KCl cardioplegic fluid without blood.

In some embodiments, as shown in Figures 2B, 4 and 6, a wire braid or coil 60 may be embedded in the wall of shaft 22 to enhance radial rigidity and to maintain the transverse dimensions of first inner lumen 29. It is particularly important to maintain the roundness of first inner lumen 29 where surgical tools are to be introduced through the first inner lumen. If shaft 22 is made of sufficient diameter to accommodate such tools through lumen 29, the shaft may tend to flatten or kink when advanced into the curved region of the aortic arch. The use of wire braid or coil 60 to maintain lumen roundness allows tool profile to be maximized and allows tools to be advanced through the lumen with minimum interference. Wire braid or coil 60 may be formed of stainless steel or other biocompatible material such as nickel titanium alloy, aramid fibers such as Kevlar™ (DuPont), or nylon.

Shaft 22 may be constructed of any of a variety of materials, including biocompatible polymers such as polyurethane, polyvinyl chloride, polyether block amide, or polyethylene. In a preferred embodiment of the device shown in Figure 2A, shaft 22 is urethane with a shore durometer in the range of 50D-80D. In the embodiment of Figure 2B, wherein shaft 22 may have a significantly larger diameter as well as an embedded coil which both increase stiffness, a polyurethane with shore durometer of 60A-100A may be used. Shaft 22 may have a bending modulus in the range of 482 to 690 MPa (70 to 100 kpsi), preferably about 551 to 621 MPa (80-90 kpsi). A bending modulus in this range provides sufficient stiffness to optimize pushability from a femoral or iliac artery to the ascending aorta, while providing sufficient flexibility to navigate the tortuous iliac artery and the aortic arch. Once partitioning device 20 has been positioned with distal end 24 in the ascending aorta, this bending modulus also facilitates exertion of a distally-directed force on shaft 22 from proximal end 26 to maintain the position of balloon 30 against the outflow of blood from the left ventricle as the balloon is inflated. In other embodiments, the dimensions, geometry and/or materials of shaft 22, as well as coil 60, may be varied over the length of the shaft so that the shaft exhibits variable bending stiffness in various regions. For example, preshaped distal portion 32 may be more flexible for tracking through the aortic arch, whereas proximal portion 36 may be stiffer for pushability and resistance to displacement.

Balloon 30 may be constructed of various materials and in various geometries. In a preferred embodiment, balloon 30 has a collapsed profile small enough for introduction into the femoral or iliac artery, e.g. 4-9 mm outside diameter, and an expanded (inflated) profile large enough to completely occlude the ascending aorta, e.g. 20-40 mm outside diameter. The ratio of expanded profile diameter to collapsed profile diameter will thus be between 2 and 10, and preferably between 5 and 10. The balloon is further configured to maximize contact of the working surface of the balloon with the aortic wall to resist displacement and to minimize leakage around the balloon, preferably having a working surface with an axial length in the range of about 3 to about 7 cm when the balloon is expanded. Textural features such as ribs, ridges or bumps may also be provided on the balloon working surface for increased frictional effects to further resist displacement.

Balloon 30 preferably has some degree of radial expansion or elongation so that a single balloon size may be used for aortas of various diameters. Materials which may be used for balloon 30 include polyurethanes, polyethylene terepthalate (PET), polyvinyl chloride (PVC), latex, ethylene vinyl acetate (EVA) and the like. However, balloon 30 must have sufficient structural integrity when inflated to maintain its general shape and position relative to shaft 22 under the systolic pressure of blood flow through the ascending aorta. In an exemplary embodiment, balloon 30 is constructed of polyurethane or a blend of polyurethane and polyvinyl such as PVC or EVA. It has been found that such materials have sufficient elastic elongation to accommodate a range of vessel diameters, while having sufficient structural integrity to maintain their shape and position in the ascending aorta when subject to outflow of blood from the left ventricle.

In a preferred embodiment, balloon 30 is further provided with a plurality of folds or pleats 62, shown in Figures 3 and 4, which allow the balloon to be collapsed by evacuation to a small collapsed profile for introduction into a femoral or iliac artery. In this embodiment, balloon 30 has a blow-up ratio, defined as the ratio of the fully-inflated outside diameter to the deflated outside diameter (before collapsing), of about 200%-400%, preferably 300%-400%. Pleats 62 are preferably at least three in number and each have a width representing approximately 5-25% of the circumference of the balloon when deflated (but not collapsed by subjecting the interior of the balloon to a vacuum). Pleats 62 may be formed into the balloon during the balloon-making process by using a dipping mandrel having longitudinal flutes formed in its periphery. The mandrel is dipped into a container of liquefied balloon material (e.g. polyurethane) so that a tubular layer of material solidifies onto the mandrel, conforming to the shape of the flutes. The mandrel is then removed, producing a pleated balloon of substantially constant thickness. Where a folded, rather than pleated, balloon is used, the folds may be formed after the balloon is made by vacuum collapsing the balloon onto a mandrel into the desired collapsed profile and heating the balloon, or by expanding the balloon under pressure and heat in a corrugated mold.

In alternative embodiments, occluding means 28 may comprise any of a variety of structures, including pivoting disk, umbrella or fan-type occlusion mechanisms actuated by pull wire, push rod, torque cable, or other type of mechanical, hydraulic, electric, or shape-memory actuator.

Referring again to Figure 1, a triple-arm adapter 64 is attached to the proximal end 26 of shaft 22. Triple-arm adapter 64 includes a working port 66 in communication with first inner lumen 29 through which straightening element 40, guidewire 42, and in some embodiments, surgical or diagnostic instruments may be introduced, as described below. Working port 66 may also be adapted for infusion of fluid such as cardioplegic fluid, saline or contrast solution, as well as for aspiration of blood, fluids and debris through first inner lumen 29. Triple-arm adapter 64 further includes an inflation port 68 in communication with the inflation lumen and configured for connection to an inflation fluid delivery device such as a syringe 70. A pressure measurement port 72 is in communication with the third lumen (48 or 54) and is adapted for connection to a pressure measurement device. Alternatively, where shaft 22 includes only first inner lumen 29 and inflation lumen 58 as in Figures 2B, 4 and 6, port 72 may be in communication with first inner lumen 29 and configured for pressure measurement, fluid infusion or aspiration.

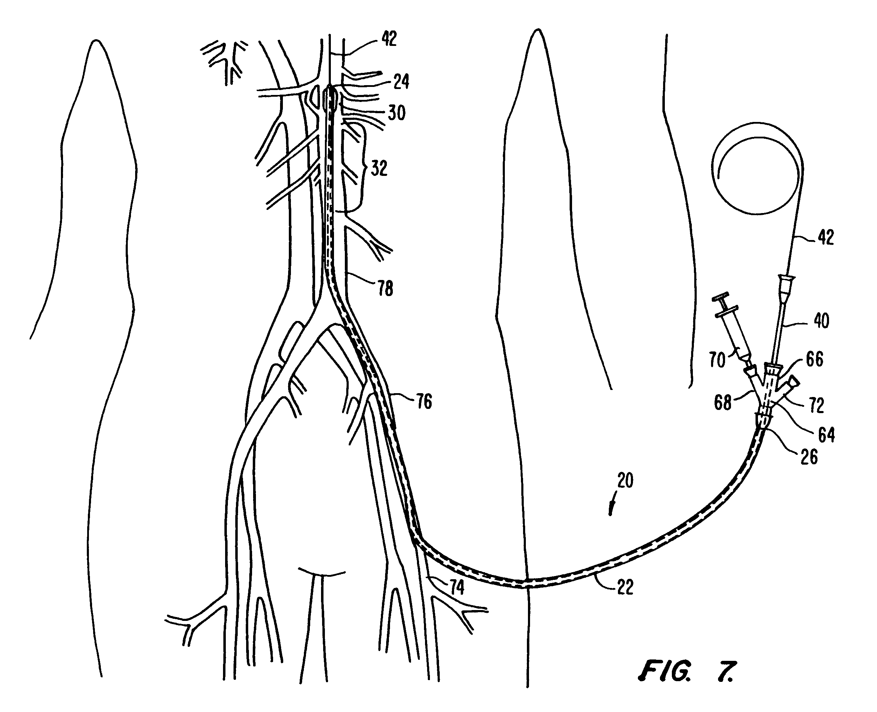

Referring now to Figures 7-9, an example of a method of using the devices of the invention will be described. Initially, a partitioning device 20 of a size and configuration suitable for the particular patient must be selected. Usually, the patient's aorta will be observed by means of a fluoroscopic imaging to determine its size and shape, particularly in the region of the aortic arch. A partitioning device 20 will be selected having a length sufficient to allow occluding means 28 to be advanced into the ascending aorta from the point of introduction, which will preferably be a femoral or iliac artery in the groin area. Further, a partitioning device will be selected which has a preshaped distal portion 32 with dimensions and shape suitable for positioning the distal portion in the patient's aortic arch such that distal end 24 is spaced apart from the inner wall of the ascending aorta, preferably aligned with the center of the aortic arch. Usually, the preshaped distal portion will have a radius of curvature approximately equal to that of the aortic arch as measured to the center of the aorta, preferably within a tolerance of about +/- 10 mm.

Referring to Figure 7, partitioning device 20 is preferably subcutaneously inserted into a femoral or iliac artery 74 in the groin area using known techniques such as a cut-down or a percutaneous technique such as the Seldinger technique. Guidewire 42 is first introduced into femoral artery 74 and advanced toward the heart through iliac artery 76 and aorta 78 so that the distal end of guidewire 42 is in the ascending aorta (not shown in Figure 7). Straightening element 40 is inserted into lumen 29 of shaft 22 and positioned in preshaped distal portion 32 so as to straighten the preshaped distal portion. With balloon 30 deflated, shaft 22 is positioned over guidewire 42, introduced into femoral artery 74 and advanced over guidewire 42 through iliac artery 76 and aorta 78. A fluoroscope may be used for visualization of radiopaque markers 39 on shaft 22 to facilitate positioning. As an alternative or supplement to fluoroscopic imaging, ultrasonic echocardiography may be used by, for example, positioning an echocardiographic transducer in the esophagus.

As an alternative to femoral or iliac introduction, shaft 22 may be introduced into carotid artery 87 or brachial artery 89. In such cases, distal portion 32 of shaft 22 will usually have a generally S-shaped configuration, as described above with reference to Figure 1C. Such an S-shaped configuration facilitates positioning balloon 30 in the ascending aorta with shaft 22 extending superiorly from the aortic arch through brachiocephalic artery 86.

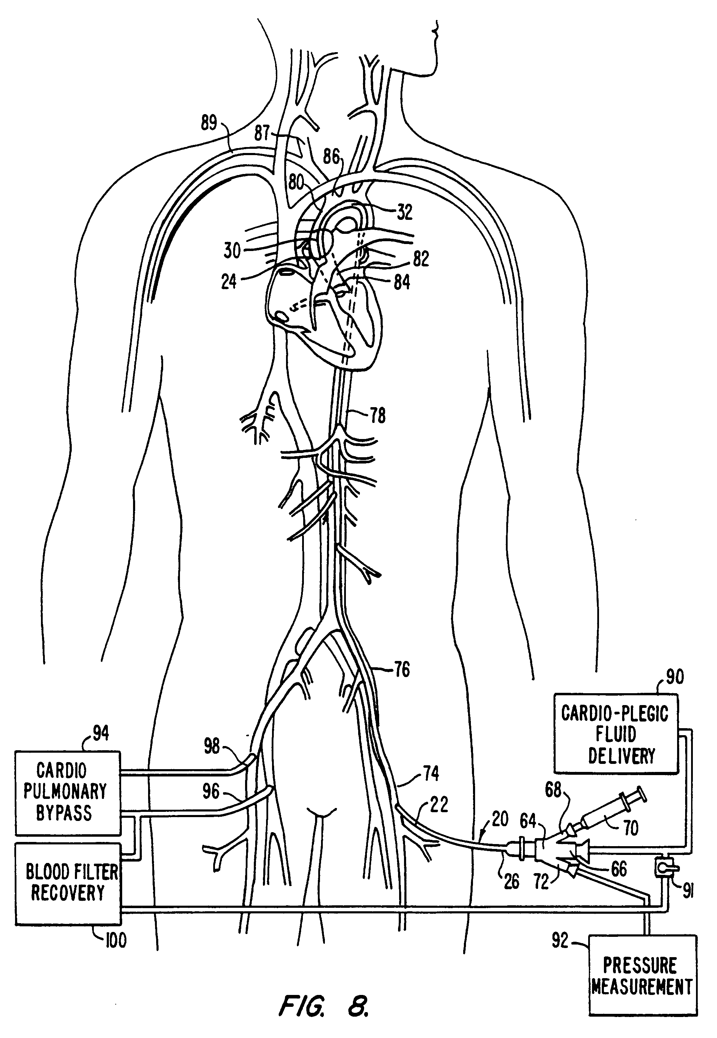

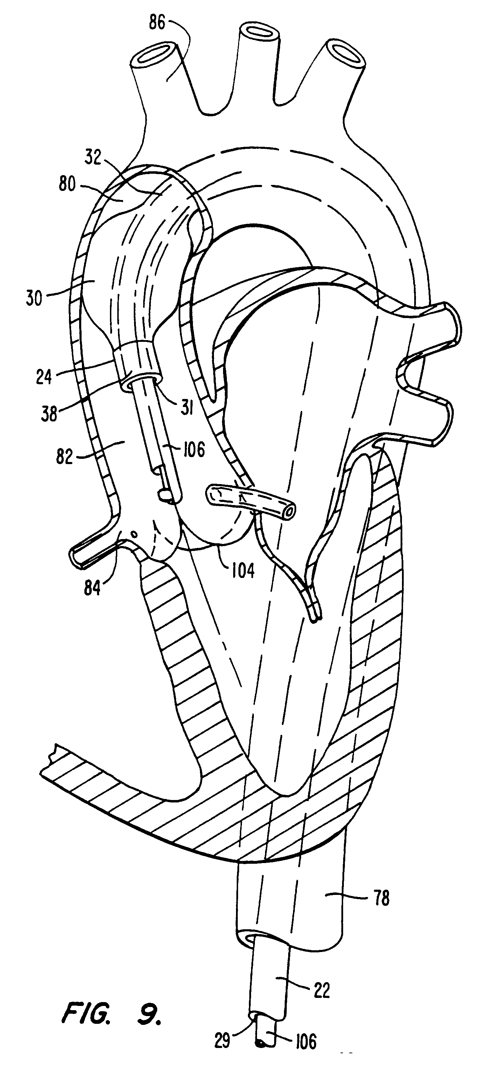

As illustrated in Figures 8 and 9, shaft 22 is advanced through aortic arch 80 until balloon 30 resides in ascending aorta 82 between coronary ostia 84 and brachiocephalic artery 86. As distal end 24 is advanced around the aortic arch, straightening element 40 is drawn proximally relative to shaft 22 so as to allow preshaped distal portion 32 to conform to the shape of the arch. In an alternative embodiment, a relatively stiff guidewire may be used without a separate straightening element, in which case the guidewire may remain in place as shaft 22 is advanced into the asending aorta. Straightening element 40 and guidewire 42 may then be removed from shaft 22.

In an alternative technique of use, partitioning device 20 may be introduced into the aorta thoracoscopically. In this case, distal end 24 of shaft 22 may be introduced through a small incision or cannula into the chest cavity. A small penetration is made in the aorta, either in the descending region or in the aortic arch. Shaft 22 is then inserted into the aorta using forceps or other thoracoscopic instruments introduced into the chest cavity through small incisions or cannulae. Such a technique may be useful where a patient's femoral or iliac arteries are unsuitable for introducing partitioning device 20 percutaneously or by cut down into those vessels.

As illustrated in Figure 8, once shaft 22 has been positioned so that balloon 30 is in ascending aorta 82 between coronary ostia 84 and brachiocephalic artery 86, balloon 30 is expanded by injecting an inflation fluid, usually a saline solution with a radiographic contrast agent, from syringe 70 through inflation port 68. In an exemplary embodiment, the balloon will be fully inflated in approximately 5-15 seconds, depending upon the size of the inflation lumen and the viscosity of the inflation fluid used. In some cases, blood may be allowed to flow through inner lumen 29 and directed to cardiopulmonary bypass system 94 (described below), thereby reducing the pressure of blood flow against balloon 30 during inflation. When fully inflated, the exterior surface of balloon 30 contacts the inner walls of the ascending aorta so as to fully occlude the vessel and block substantially all systolic and diastolic blood flow past the balloon. While the heart remains beating, blood may flow from the left ventricle through the aortic valve and into the coronary ostia so as to perfuse the myocardium through the coronary arteries. The heart and coronary arteries are thus isolated from the remainder of the arterial system.

In an alternative technique of use, a gaseous inflation fluid may be used in order to increase inflation speed. In this way, balloon 30 can be fully inflated in less time than the period between systolic pulses, reducing the likelihood that the outflow of blood from the left ventricle during systole will displace balloon 30 from its position in the ascending aorta. Preferably, carbon dioxide is used as the inflation fluid, since carbon dioxide, being highly soluble in blood, is unlikely to produce potentially injurious gas emboli in the event of leakage from the balloon. Alternatively, helium may be used. A gas inflation pump and control device similar to those described in U.S. Patent No. 4,771,765 and U.S. Patent No. 4,902,272 may be utilized for delivery of pressurized gas through inflation port 68. The inflation pump may be timed with the contractions of the heart to facilitate inflation of the balloon between systolic pulses. Using such a pump, balloon 30 may be fully inflated in less than about 1 second, and preferably less than about 0.5 second.

Figure 8 illustrates the components of a system for arresting the heart constructed in accordance with the principles of the invention. A cardioplegic fluid delivery device 90 is connected to working port 66. A pressure measurement device 92 may be connected to port 72 to monitor pressure in the ascending aorta upstream of balloon 30 through a lumen in shaft 22. The patient is placed on a cardiopulmonary bypass (CPB) system 94 to maintain circulation of oxygenated blood throughout the body. Usually, a venous cannula 96 is positioned in a femoral vein for withdrawing de-oxygenated blood. In addition, a pulmonary artery venting catheter (not shown) may be positioned through the right internal jugular vein into the pulmonary trunk to withdraw the blood contained therein, thereby decompressing the left atrium. The withdrawn blood is delivered to CPB system 94 which removes carbon dioxide and oxygenates the blood. The oxygenated blood is then delivered to a femoral or iliac artery via an arterial cannula 98. A blood filter and recovery system 100 may also be connected to port 66 in partitioning device 20 to receive blood and other fluids and debris from first inner lumen 29, filter the blood to remove impurities, and deliver the blood to CPB system 94 for return to the patient's circulatory system. A valve 91 may be provided to select between cardioplegic fluid delivery or venting of fluids through working port 66. Further aspects of a CPB system suitable for use in the system of the invention are described in F. Rossi et al., *Long-Term Cardiopulmonary Bypass By Peripheral Cannulation In A Model of Total Heart Failure,* Journal of Thoracic and Cardiovascular Surgery (1990), 100:914-921.

With CPB established and balloon 30 blocking blood flow through the ascending aorta, the myocardium may then be paralyzed. In a preferred embodiment, a fluid containing cardioplegic agents is delivered by delivery device 90 through working port 66. The cardioplegic fluid preferably consists of an aqueous KCl solution mixed with oxygenated blood at a ratio of four parts blood to one part KCl solution. The aqueous KCl solution consists of crystalloid KCl mixed with saline to have a concentration in the range of 10-50 mEq K⁺/liter, preferably 15-30 mEq K⁺/liter. Delivery device 90 includes a cooler such as an ice bath (not shown) which cools the cardioplegic fluid to e.g. 3°C-10°C, so as to maintain the heart at a low temperature and to minimize demand for oxygen. This is usually accomplished without applying external cooling to the heart as is generally applied in conventional open cardiac procedures. The cardioplegic fluid is infused into the ascending aorta through opening 31 at the distal end of partitioning device 20 to maintain a pressure in the aortic root distal to balloon 30 sufficient to induce flow of fluid into the coronary arteries through coronary ostia 84. A pressure of about 8,00 - 9,33 KPa (60-80 mmHg) as measured through third lumen 48 is usually sufficient. Cardioplegic fluid is preferably delivered at a flowrate of about 250-350 ml/min. so as to deliver a total volume of 750-1000 ml in about 2-4 minutes, although this may vary depending upon patient anatomy, physiological changes such as coronary dilation, and other factors. In pumping the cardioplegic fluid through inner lumen 29, the fluid should be subject to a pump pressure of no more than about 40,00 KPa (300 mmHg) to minimize damage to the blood component of the mixture. Cardioplegic fluid may also be infused in a retrograde manner through the coronary sinus, by means of a catheter (not shown) positioned transluminally through the right internal jugular vein, as described above. Heart contractions will then cease, with circulation to the remainder of the patient's body maintained by CPB system 94. Cardioplegic fluid flow to the patient's myocardium is maintained on a periodic basis, e.g., about every 10--20 minutes for 2-4 minutes, so long as the myocardium is to remain paralyzed. A comprehensive description of cardioplegic techniques suitable for use in the method of the invention is found in Buckberg, *Strategies and logic of cardioplegic delivery to prevent, avoid, and reverse ischemic and reperfusion damage,* J. Thorac. Cardiovasc. Surg. 1987;93:127-39.

In addition to or instead of infusion of the blood/crystalloid cardioplegic solution, other techniques may be used to arrest heart contractions. A more concentrated crystalloid KCl solution not mixed with blood may be delivered through inner lumen 29 at higher pressures than with a blood cardioplegic fluid mixture, since without blood in the solution, there is no risk of hemolysis. This allows inner lumen 29 (as well as catheter shaft 22) to be of smaller cross-sectional area while still providing the necessary flowrate of fluid into the aortic root. However, the above blood cardioplegia technique is presently preferred because it is generally believed to provide greater myocardial protection. In another alternative technique, the patient's body may be cooled in a cold-temperature environment or by application of cold-packs to the chest to reduce the temperature of the myocardium sufficiently to induce fibrillation. The myocardium may be cooled directly by infusion of cold fluid such as cold blood or saline through the coronary arteries. Alternatively, electrical fibrillation may be accomplished by delivering electrical signals to the myocardium by means of electrodes placed on the exterior surface of the heart or externally on the chest. However, cardiac arrest by means of fibrillation is generally less desirable than chemical cardioplegic paralysis because there remains some degree of heart motion which could make surgical intervention more difficult and because there is a significantly higher demand for oxygen, reducing the safety and duration of the procedure.

Once the heart has been arrested and CPB established, a surgical procedure may be performed. The procedure will preferably be a less-invasive procedure performed endovascularly or thoracoscopically, as described in US-A-5452733 (application Serial No. 08/023,778).
Surgical procedures which may be performed using the device and system of the invention include repair or replacement of the aortic, mitral and other heart valves, repair of ventricular and atrial septal defects, septal myotomy, cardiac mapping and ablation to correct arrhythmias, coronary artery bypass grafting, angioplasty, atherectomy, as well as pulmonary, neurosurgical, and other procedures.

Partitioning device 20 of the present invention is particularly advantageous for endovascular introduction of surgical instruments through the aorta for procedures such as heart valve repair and replacement. As illustrated in Figure 9, preshaped distal portion 32 of shaft 22 conforms to the shape of aortic arch 80 so that opening 31 at the distal end is positioned centrally within the ascending aorta and axially aligned with the center of aortic valve 104. This not only enhances infusion of cardioplegic fluid through opening 31, but ensures that surgical instruments such as valve cutter 106 introduced through first inner lumen 29 will be aligned with aortic valve 104, either to remove the valve, or to pass through it for intracardiac procedures. Advantageously, soft tip 38 at the distal end of shaft 22 prevents damage to tissue, particularly the fragile aortic valve leaflets, in the event of contact therewith.

While being particularly useful in conjunction with minimally-invasive cardiac procedures performed endovascularly and/or thoracoscopically, the partitioning device and system for arresting the heart disclosed herein are also useful in conventional open procedures performed with a thoracotomy. Partitioning device 20 may be used where an aortic cross-clamp would pose risks of embolus release due to calcification or other aortic conditions. In open procedures, partitioning device 20 may be introduced through the femoral or iliac arteries as described above, through the carotid artery 87, through the brachial artery 89, or through a penetration in the aorta itself, which is accessible as a result of the thoracotomy. In such cases, shaft 22 of partitioning device 20 may be substantially shorter in length, for example, 20 to 60 cm.

When the procedure has been completed, the heart is restarted by discontinuing any flow of cardioplegic fluid through partitioning device 20 or retrogradely through the coronary sinus, ventilating the lungs, and perfusing the coronary arteries with warm blood. The region upstream of balloon 30 may be irrigated by infusing a saline solution through first inner lumen 29. Blood and other fluids upstream of balloon 30 may then be aspirated through first inner lumen 29 to remove thrombi or other emboli which may have been produced during the procedure, preventing such emboli from entering the brachiocephalic, carotid, or subclavian arteries and greatly reducing the risk of complications such as strokes. Balloon 30 is deflated to allow normal flow of warm blood through the ascending aorta to the remainder of the arterial system. Normal heart contractions may resume promptly, or, if necessary, electrical defibrillation may be administered to correct heart rhythm. CPB is gradually discontinued, and CPB venous cannula 96 and arterial cannula 98 are removed. Partitioning device 20 is withdrawn from the body back through the site of entry, and the arterial penetration is closed. If the patient has been put under general anesthesia, the patient is then brought from anesthesia to consciousness.

It will be understood by those of skill in the art that various alternative configurations of endovascular partitioning device 20 are possible without departing from the scope of the present invention as claimed.

The partitioning device and system for arresting the heart disclosed herein offer significant advantages over current techniques. Primary among these advantages is the ability to block blood flow through the ascending aorta and stop heart contractions using an endovascular device, without the need for a thoracotomy. By obviating the need to open the chest for external clamping of the aorta, the invention facilitates the performance of a new generation of minimally-invasive cardiac and vascular procedures. Elimination of a thoracotomy in such procedures produces lower mortality and morbidity, reduced patient suffering, decreased hospitalization and recovery time, and reduced medical costs. Moreover, the invention is useful even in open-chest procedures as a substitute for the aortic cross-clamp where calcification or other conditions could make external aortic clamping undesirable.

## Claims

1. An endovascular device (20) for partitioning a patient's ascending aorta between the coronary ostia and the brachiocephalic artery, comprising:
a flexible shaft (22) having a distal end (24), a proximal end (26), a first inner lumen (29) extending therebetween, an opening (31) at the distal end in fluid communication with the first inner lumen, and a distal portion (32) positionable within the ascending aorta (82) from an arterial location downstream thereof; and
expandable means (28) near the distal end (26) of the shaft proximal to the opening (31) in the first inner lumen for occluding the ascending aorta (82) between the coronary ostia (84) and the brachiocephalic artery (86) so as to block substantially all blood flow therethrough,
**characterized in that** the distal portion (32) is preshaped to conform generally to a patient's aortic arch and there are means (40) for straightening the distal portion (32) of the shaft (22) to facilitate introducing the shaft into an artery downstream of the patient's ascending aorta.

2. The endovascular device of claim 1 wherein the distal portion (30) is generally U-shaped in an unstressed condition.

3. An endovascular device according to claim 1 wherein the distal portion (32) includes at least one bend (33) configured to engage an inner aortic wall.

4. An endovascular device according to claim 1 wherein the distal portion (32) is generally S-shaped in an unstressed condition.

5. An endovascular device according to any one of the preceding claims wherein the straightening means (40) comprises a movable guidewire (42) extending through the first inner lumen.

6. An endovascular device according to any one of claims 1-4 wherein the straightening means (40) comprises a stylet slidably disposed in an inner lumen of the shaft.

7. An endovascular device according to any one of the preceding claims wherein the occluding means comprises an inflatable balloon with an interior for receiving an inflation fluid for the inflation thereof.

8. An endovascular device according to claim 7 further comprising a second lumen (46,56,58) extending through the shaft from the proximal end to the interior of the balloon (30) for inflation thereof.

9. An endovascular device according to claim 8 further comprising a third lumen (48,54) extending from the proximal end to the distal end of the shaft, the third lumen having an opening distal to the occluding means.

10. An endovascular device according to claim 9 further comprising means in fluid communication with the third lumen for sensing pressure distal to the occluding means through the third lumen.

11. An endovascular device according to any one of the preceding claims wherein the shaft has a length of at least about 80 cm to allow transluminal positioning of the shaft from a femoral artery to the ascending aorta.

12. A system for arresting a patient's heart comprising an endovascular device according to any one of the preceding claims; and
means for paralyzing the patient's myocardium.

## Patentansprüche

1. Endovaskulare Vorrichtung (20) zum Unterteilen der aufsteigenden Aorta eines Patienten zwischen den Koronarostia und der Brachiozephalarterie, umfassend:
einen flexiblen Schaft (22), der ein distales Ende (24), ein proximales Ende (26), ein sich zwischen diesen erstreckendes erstes Innenlumen (29), eine Öffnung (31) am distalen Ende in Fluidkommunikation mit dem ersten Innenlumen sowie einen distalen Abschnitt (32) umfasst, der innerhalb der aufsteigenden Aorta (82) von einer arteriellen Position stromab davon angeordnet werden kann; sowie
ein erweiterbares Mittel (28) nahe dem distalen Ende (26) des ersten Schafts proximal zur Öffnung (31) im ersten Innenlumen, um die aufsteigende Aorta (82) zwischen den Koronarostia (84) und der Brachiozephalarterie (86) zu verschließen, um im Wesentlichen den gesamten Blutstrom durch sie hindurch zu blockieren,
**dadurch gekennzeichnet, dass** der distale Abschnitt (32) so vorgeformt ist, dass er im Wesentlichen dem Aortabogen eines Patienten entspricht und Mittel (40) vorhanden sind, um den distalen Abschnitt (32) des Schafts (22) gerade zu richten, um das Einführen des Schafts in eine Arterie stromab von der aufsteigenden Aorta des Patienten zu erleichtern.

2. Endovaskulare Vorrichtung nach Anspruch 1, worin der erste Abschnitt (30) in einem unbelasteten Zustand allgemein U-förmig ist.

3. Endovaskulare Vorrichtung nach Anspruch 1, worin der distale Abschnitt (32) zumindest eine Biegung (33) umfasst, die eine solche Konfiguration aufweist, dass sie an einer Aorta-Innenwand angreift.

4. Endovaskulare Vorrichtung nach Anspruch 1, worin der distale Abschnitt (32) in einem unbelasteten Zustand allgemein S-förmig ist.

5. Endovaskulare Vorrichtung nach einem der vorangegangenen Ansprüche, worin das Mittel (40) zum Geraderichten einen beweglichen Führungsdraht (42) umfasst, der sich durch das erste Innenlumen erstreckt.

6. Endovaskulare Vorrichtung nach einem der Ansprüche 1 bis 4, worin das Mittel (40) zum Geraderichten einen Katheterdrain umfasst, der gleitend in einem Innenlumen des Schafts angeordnet ist.

7. Endovaskulare Vorrichtung nach einem der vorangegangenen Ansprüche, worin das Verschlussmittel einen aufblasbaren Ballon mit einem Innenraum zum Aufnehmen eines Aufblasfluids zum Aufblasen desselben umfasst.

8. Endovaskulare Vorrichtung nach Anspruch 7, weiters umfassend ein zweites Lumen (46, 56, 58), das sich vom proximalen Ende durch den Schaft zum Innenraum des Ballons (30) erstreckt, um ihn aufzublasen.

9. Endovaskulare Vorrichtung nach Anspruch 8, weiters umfassend ein drittes Lumen (48,54), das sich vom proximalen Ende zum distalen Ende des Schafts erstreckt, wobei das dritte Lumen eine zum Verschlussmittel distale Öffnung aufweist.

10. Endovaskulare Vorrichtung nach Anspruch 9, weiters umfassend Mittel in Fluidkommunikation mit dem dritten Lumen zum Abfühlen von Druck distal zum Verschlussmittel durch das dritte Lumen.

11. Endovaskulare Vorrichtung nach einem der vorangegangenen Ansprüche, worin der Schaft eine Länge von zumindest etwa 80 cm aufweist, um transluminale Positionierung des Schafts von einer Oberschenkelarterie zur aufsteigenden Aorta zu ermöglichen.

12. System, um das Herz eines Patienten zum Stillstand zu bringen, umfassend eine endovaskulare Vorrichtung nach einem der vorangegangenen Ansprüche; und
Mittel zum Paralysieren des Herzmuskels des Patienten.

## Revendications

1. Dispositif endovasculaire (20) pour séparer l'aorte ascendante d'un patient entre les orifices coronaires et le tronc artériel brachio-céphalique, comprenant :
- une tige flexible (22) présentant une extrémité distale (24), une extrémité proximale (26), une première lumière interne (29) s'étendant entre celles-ci, une ouverture (31) à l'extrémité distale en communication de fluide avec la première lumière interne, et une portion distale (32) pouvant être positionnée dans l'aorte ascendante (82) à partir d'un emplacement artériel en aval de celle-ci ; et
- un moyen expansible (28) près de l'extrémité distale (26) de l'arbre proximal à l'ouverture (31) dans la première lumière interne pour fermer l'aorte ascendante (82) entre les orifices coronaires (84) et le tronc artériel brachio-céphalique (86) de façon à bloquer sensiblement tout écoulement de sang à travers celle-ci,
**caractérisé en ce que** la portion distale (32) est formée préalablement pour s'adapter généralement à l'arc de l'aorte du patient , et il y a des moyens (40) pour redresser la portion distale (32) de l'arbre (22) pour faciliter l'introduction de la tige dans une artère en aval de l'aorte ascendante du patient.

2. Dispositif endovasculaire selon la revendication 1, où la portion distale (30) a généralement une forme en U à l'état non sollicité.

3. Dispositif endovasculaire selon la revendication 1, où la portion distale (32) comprend au moins un pliage (33) configuré pour s'engager dans une paroi aortique interne.

4. Dispositif endovasculaire selon la revendication 1, où la portion distale (32) est généralement en forme de S à l'état non sollicité.

5. Dispositif endovasculaire selon l'une des revendications précédentes, dans lequel le moyen de redressement (40) comprend un fil de guidage mobile (42) s'étendant à travers la première lumière intérne.

6. Dispositif endovasculaire selon l'une des revendications 1 à 4, où le moyen de redressement (40) comprend un stylet disposé d'une manière coulissante dans une lumière interne de la tige.

7. Dispositif endovasculaire selon l'une des revendications précédentes, où le moyen d'occlusion comprend un ballon gonflable avec un intérieur pour recevoir un fluide de gonflement en vue du gonflement de celui-ci.

8. Dispositif endovasculaire selon la revendication 7, comprenant en outre une deuxième lumière (46, 56, 58) s'étendant à travers la tige de l'extrémité proximale à l'intérieur du ballon (30) pour le gonflement de celui-ci.

9. Dispositif endovasculaire selon la revendication 8, comprenant en outre une troisième lumière (48, 54) s'étendant de l'extrémité proximale à l'extrémité distale de l'arbre, la troisième lumière ayant une ouverture distale au moyen d'occlusion.

10. Dispositif endovasculaire selon la revendication 9, comprenant en outre un moyen en communication de fluide avec la troisième lumière pour détecter la pression distale au moyen d'occlusion à travers la troisième lumière.

11. Dispositif endovasculaire selon l'une des revendications précédentes, dans lequel l'arbre a une longueur d'au moins environ 80 cm pour permettre un positionnement transluminal de l'arbre d'une artère fémorale à l'aorte ascendante.

12. Système pour arrêter le coeur d'un patient, comprenant un dispositif endovasculaire selon l'une des revendications précédentes ; et
- un moyen pour paralyser le myocarde du patient.
